# EUROPEAN PATENT SPECIFICATION

(11) **EP 0 783 891 B1**
(45) Date of publication and mention of the grant of the patent: **27.12.2006**
(21) Application number: 96925093.5
(22) Date of filing: 25.07.1996
(51) Int. Cl.: A61K 38/21, A61K 45/00, A61P 19/08, A61P 19/10

(54) **INTERFERON-BETA FOR BONE DISEASES**
INTERFERON-BETA GEGEN KNOCHENERKRANKUNGEN
INTERFERON-BETA CONTRE LES MALADIES OSSEUSES

(30) Priority: 25.07.1995 JP 18897295
(43) Date of publication of application: 16.07.1997
(60) Divisional application: 04001847.5
(73) Proprietor: TORAY INDUSTRIES, INC., Tokyo 103-8666 (JP)
(72) Inventor: IDA, Nobutaka, Kanagawa 248 (JP); SUZUKI, Tomohiko, Kanagawa 215 (JP); KUMAGAI, Emi, Kanagawa 248 (JP)
(74) Representative: Kador & Partner
(86) International application number: PCT/JP1996/002099
(87) International publication number: WO 1997/004799

(56) References cited:
- JP-A- 7 215 893
- JP-A- 62 012 724
- HAASE K.D. ET AL: "Local interferon treatment of osteolytic bone metastases." TUMOR DIAGNOSTIK UND THERAPIE, (1988) 9/3 (96-99)., XP000918861
- CAMPISI C ET AL: "Complete resolution of breast cancer bone metastasis through the use of beta - interferon and tamoxifen." EUROPEAN JOURNAL OF GYNAECOLOGICAL ONCOLOGY, (1993) 14 (6) 479-83., XP000918023
- NILSSON O.S. ET AL.: "Effect of interferon on heterotopic new bone formation in mice" J. INTERFERON RES., vol. 4, no. 1, 1984, pages 135-140, XP000940501
- DATABASE WPI Section Ch, Week 199217 Derwent Publications Ltd., London, GB; Class B04, AN 1992-136707 XP002151127 & JP 04 077431 A (KANEBO LTD), 11 March 1992 (1992-03-11)
- DATABASE WPI Section Ch, Week 198220 Derwent Publications Ltd., London, GB; Class B04, AN 1982-40146E XP002151128 & JP 57 058630 A (KAYAKU ANTIBIOTICS RES CO), 8 April 1982 (1982-04-08)
- GOWEN M. ET AL: "Studies on the actions of interleukin-1 on bone metabolism: IL-1 stimulation of bone cell proliferation, and inhibition of IL-1-induced bone resorption by interferon -. gamma.." BRITISH JOURNAL OF RHEUMATOLOGY, (1985) 24/SUPPL. 1 (147-149). CODEN: BJRHDF, XP001056335
- CALCIFIED TISSUE INTERNATIONAL, Vol. 53, Suppl. 1 (1993) G. DAVID ROODMAN, "Role of Cytokines in the Regulation of Bone Resorption", S94-S98, XP000940463.
- GERONTOLOGY, Vol. 35 (1989) SHUNICHI SHIOZAWA et al., "Radioimmunoassay of Circulating Alpha-Interferon with Reference to Aging and Osteoporosis", p. 305-310, XP000940503.

## Description

### TECHNICAL FIELD

The present invention relates to use of interferon β as a drug for bone disorders. The drug is used for preventing or curing bone disorders caused by various causes such as physical and metabolic causes and by malignant tumors. The present invention also includes use of interferon inducers as a drug for preventing and curing bone disorders.

### BACKGROUND ART

Bones are important tissues in vertebrates, which support the body. Unlike the image from their outer appearances, bones actively repeat degradation of bones by osteoclasts and formation of bones by osteoblasts, thereby attaining the homeostasis of their morphology and physical strength based on the exquisite balance therebetween. If this balance is lost, various bone disorders are caused. In recent years, osteoporosis is especially a big medical and social problem (H. Orimo and H. Ozawa, "Graphically Shown Osteoporosis", MEDICAL VIEW, 1990). Osteoporosis does not necessarily occur when the osteoclastic activity is abnormally high, but also occurs, for example, even when the osteoclastic activity is low if the bone formation is lower. Osteoporosis includes the high turnover type in which both the bone resorption and bone formation are active, and the low turnover type in which both the bone resorption and bone formation are inactive. The former is also called type I and frequently occurs among postmenopausal women, and the latter is also called type II and frequently occurs among old people irrespective of sex. Thus, what is important is the relative balance between the bone formation and the osteoclastic activity, and the balance is finally reflected to the bone volume and bone strength. Therefore, it is thought effective for therapy of bone disorders accompanying decrease in bone mineral density to relatively increase the rate of bone formation with respect to the rate of bone resorption or to relatively decrease the rate of bone resorption with respect to the rate of bone formation.

In the bone resorption process, osteoclasts play the most important role (Baron, R. et al., Bone and Mineral Res., 2, 175-243, Elsevier, New York, 1984). In addition to physiological causes such as menopause and aging, bone disorders are caused by various physical, chemical and biological causes as well as by unknown causes, which influence on the proliferation, differentiation or expression of functions of these cells so as to disturb the relative balance between the bone resorption and bone formation. The disorders in which the relative balance between the bone resorption and bone formation is disturbed include cancer-related diseases such as bone metastasis of lung cancer, mammary carcinoma or renal cancer, and multiple myeloma; metabolic bone disorders such as Paget's disease, rickets, osteomalacia, marble bone disease (osteopetrosis), osteoarthritis and osteogenesis imperfecta; diseases related to hormonal disorders, such as Cushing's syndrome and hyperparathyroidism; autoimmune disorders such as rheumatoid arthritis; and periodontal diseases such as alveolar bone disorder. Osteoblasts are mononucleated cells differentiated from immature mesenchymal cells originated from mesoderm. Since osteoblasts separated from bone tissues of experimental animals have the property to divide and proliferate *in vitro*, a number of cell lines have been established. Examples of such established cell lines include MC3T3-E1 (Sudo, H. et al., J. Cell Biol., 96, 191, 1983), ROS17/2, UMR106 (Fraser, J.D. et al., J. Biol. Chem., 263, 911, 1988) and RCT-3 (Hearth, J. K. et al., Endocrinology, 124, 3060, 1988).

Osteoclasts are multinucleated giant cells (Wergedal, J. E., Proc. Soc. Exp. Biol. Med., 134, 244, 1970, Clark, S.E. et al., J. Bone Mineral Res., 4, 399, 1989), having the features that they have calcitonin receptors (Warshawsky, H. D. et al., J. Cell Biol., 85, 682, 1980, Nicholson, G. C. et al., J. Clin. Invest., 78, 355, 1986), and that in bone resorption, they ionize the calcium contained in the bone matrix and transport the calcium to body fluid (Blair, H.C. et al., Science, 245, 855, 1989). Embryologically, osteoclasts are formed by differentiation of bone marrow cells. It is known that osteoclast like cells which express the fundamental properties of osteoclasts are formed by culturing mononuclear cells of bone marrow or spleen in the presence of 1,25-dihydroxyvitamin D₃ or prostaglandin E₂ (Takahashi, N. et al., Endocrinology, 122, 1373, 1988, Udagawa, N. et al., Endocrinology, 125, 1805, 1989).

Interferon (hereinafter referred to as "IFN" for short) is a protein which exhibits various physiological activities including antiviral and antitumor activities. Interferon is now classified into α, β and γ types depending on the differences in history of discovery, structure and physicochemical properties. For example, IFNα is mainly produced by leukocytes, lymphoblastoid cells, NK cells, B cells and the like; IFNβ is mainly produced by fibroblast cells; and IFNγ is mainly produced by T cells and NK cells. All of these types of IFN are now commercially available as an antitumor agent or an agent for treating hepatitis and clinically used for human. Since administration of 1,25-dihydroxyvitamin D₃ ' or calcitonin to patients suffering from osteoporosis increases the blood level of IFNα (Shiozawa, S. et al., Gerontol., 35, 305, 1989), some relationship was suggested (T. Fujita, Clinical Calcium, 2, 852, 1992). Inhibition of formation of osteoclast like cells by various recombinant IFNα *in vitro* in human cord blood cells has been reported (K. Anai et al., Nihon-Kotsutaisha-Gakkai Zassi (in Japanese) Vol. 9, 238, 1991). Further, it has been reported that administration of human recombinant IFNα to patients suffering from hepatitis C decreases the level of urinary deoxypyridinoline which is an index of the osteoclastic activity (H. Yoshida et al., Nihon-Kotsutaisha-Gakkai Zassi, Vol. 12, 215, 1994) or increases the bone volume (M. Kawakatsu et al., Nihon-Kotsutaisha-Gakkai Zassi, Vol. 12, 234, 1994). On the other hand, however, it has also been reported that administration of human recombinant IFNα to patients suffering from hepatitis C does not influence on the bone volume of the patients at all (T. Tanaka et al., Japanese Journal of Gastroenterology, Vol. 91, Subject 0-423, 1994). Thus, evaluation of IFNα is in chaos. The chaotic state about the existence of the effects is also seen in the basic studies. That is, it has been reported that murine IFN inhibits bone resorption *in vitro* induced by parathyroid hormone in an organ culture system of bone (Jilka, R. L. and Hamilton, J. W., Biochem. Biophys. Res. Commun., 120, 553, 1984). On the other hand, it has also been reported that administration of murine IFN to mice in which decalcified bone matrix was subcutaneously transplanted gives no effects at all *in vivo* (Nilsson, O.S. et al., J. Interferon Res., 4, 135, 1984). The IFN used in both of these experiments was a mixture of IFNα and β. Further, it has been reported that IFNα is not effective against rheumatoid arthritis at all (Kajandahl, A. et al., Lancet, Vol. i, 984, 1979) and even there is a report which insists the disadvantage of IFN, that is, which reports that IFN induces experimental synovitis and that IFN induces autoimmune diseases in human (Rosenbach, T.O. et al., Clin. Rheumatol., 3, 361-364, 1984). Thus, the evaluation of IFN per se has not been fixed and the existence of effects of IFNβ alone is not known at all.

The substantial part of the reason why it is unclear whether an effect of IFN on bone metabolism exists or not is the species specificity of IFN. Especially, unlike IFNα, IFNβ has a strong species specificity. Therefore, evaluation of the effect of IFNβ must be made using the IFNβ of the animal of the species of interest. As mentioned above, increase and decrease in bone volume is finally determined by the relative strength of the effect on the bone formation system and on the bone resorption system, the correct evaluation of IFNβ is attained only by a comparative experiment on the strictly and scientifically controlled bone resorption system and bone formation system using the IFNβ from the animal species of interest. The present inventors insist that it is indispensable to the development of a drug for bone disorders to make relative evaluations on both bone formation system and bone resorption system. The correctness of this opinion will be concretely shown by the example employing IFNγ described below.

The reports about IFNγ are most abundant among the reports about the influences by IFN on bone metabolism. Most of the reports utilize the inhibition of formation of osteoclast like cells from bone marrow cells in the experimental system established by Takahashi et al., which inhibition is attained by stimulation by bradykinin (Lerner, U.H. et al., Agents and Actions, 32, 305, 1991), forskolin, cholera toxin (Lerner, U.H. et al., J. Bone Min. Res., 6, 551, 1991), interleukin 1 (Foffmann, O. et al., Biochem. Biophys. Res. Commun., 143, 38, 1987) or by 1,25-dihydroxyvitamin D₃ (Gowen, M. et al., J. Bone Min. Res., 1, 469, 1986). Prior to these reports, the action of IFNγ on bone metabolism had already been disclosed in a patent application (M. Peterlik, Japanese Patent Application No. 61-123862). If evaluation is made based on the position that evaluation in the bone resorption system alone is sufficient, inhibition of formation of osteoclasts should reduce or stop the decrease in bone volume, or increase the bone volume since osteoclasts carry out bone resorption, so that the invention directed to the use of IFNγ as a drug for improving bone volume may be regarded as completed or may be inferred easily. However, the truth was on the contrary. It has been reported that administration of IFNγ has no therapeutic effects against osteopenia (according to Stedman's Medical Dictionary, Revised 2nd Edition, Medical View Co., Ltd., 1988, osteopenia means deposition of calcium or decrease in density of bone), but decreases the bone volume, and it was suggested that the reason therefor may be the inhibition of deposition of bone mineral (Mann, G. N. et al., Endocrinology, 135, 1077, 1994). Further, ironically, the later studies revealed that IFNγ having strong osteoclast formation-inhibition activity exhibited excellent therapeutic effect against marble bone disease against which promotion or activation of bone resorption by formation of osteoclasts is needed for therapy or relief of this disease in both an animal (Rodriguiz, R.M. et al., Pediatrics Res., 33, 384, 1993) and in human (Lyndon Key, Jr. L. et al., NewEngl. J. Med., 332, 1544, 1995).

Thus, in order to be a drug effective for therapy of bone disorders, especially the bone disorders requiring increase in the bone volume, the fact that the drug or the substance exhibits inhibition of bone resorption is insufficient, but the fact that the drug or the substance does not inhibit at all or only slightly inhibits the bone formation system (proliferation, differentiation and mineralization of osteoblasts), or preferably, the fact that the drug activates the bone formation system, must be proved. In fact, by solving the problem described below, the present inventors reached a conclusion which is contrary to the above-mentioned observation by Nilsson et al. (J. Interferon Res., 4, 135, 1984) that administration of mouse IFN is not effective at all for bone formation in mice. That is the present inventors completed the present invention by proving the effect for promoting bone formation so as to reinstate the bone volume which was once decreased in bone disorder model animals.

### DISCLOSURE OF THE INVENTION

According to the present invention, based on the relative evaluation of both the strictly controlled bone resorption system and bone formation system using IFNβ free from the ambiguity of the substance, the use of IFNβ for the manufacture of a drug for treatment of a disturbed relative balance between bone resorption and bone formation wherein the bone disorder is osteoporosis, metabolic bone disorder, hormonal bone disorder, periodontal disease-related bone disorder, bone fracture or hypercalcemia, which is useful in industry and in medicine is provided. Further, based on the discoveries during the process of completing the present invention, use of IFN β for the manufacture of a drug for treatment of a disturbed relative balance between bone resorption and bone formation wherein the bone disorder is osteoporosis, metabolic bone disorder, hormonal bone disorder, periodontal disease-related bone disorder, bone fracture or hypercalcemia inducers is provided by the present invention.

### BRIEF DESCRIPTION OF THE DRAWINGS

Fig. 1 shows inhibition actions of murine IFNβ and γ against the formation of osteoclast like cells. The bone marrow cells are originated from mice of ddy strain.
Fig. 2 shows inhibition actions of murine IFNβ and γ against the formation of osteoclast like cells. The bone marrow cells are originated from mice of C 57 BL/6 strain.

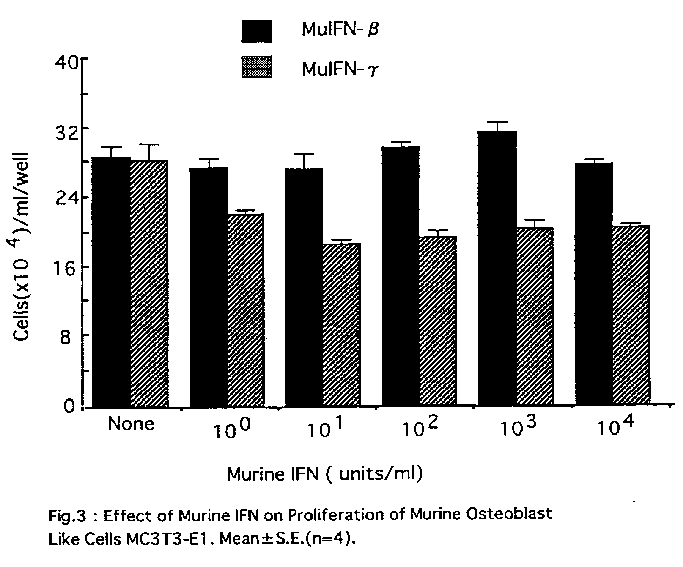

Fig. 3 shows actions of murine IFNβ and γ on the proliferation of murine osteoblastic cells MC3T3-E1.
Fig. 4 shows the difference between the actions by murine IFNβ and γ on the mineralization of murine osteoblastic cells MC3T3-E1.
Fig. 5 shows the amount of calcium extracted from the calcified foci of murine osteoblastic cells MC3T3-E1 treated with murine IFN.
Fig. 6 shows the amount of inorganic phosphorus extracted from the calcified foci of murine osteoblastic cells MC3T3-E1 treated with murine IFN.
Fig. 7 shows effect for promoting mineralization in murine osteoblastic cells MC3T3-E1 by frequent administration of murine IFNβ.
Fig. 8 shows the effect for restoring bone volume of mice by administration of IFNβ, which bone volume was decreased by ovariectomy.
Fig. 9 shows inhibition effect by administration of murine IFNβ on the bone resorption which was increased by ovariectomy.
Fig. 10 shows change in body weights of mice during the experiment of administration of murine IFNβ.

### BEST MODE FOR CARRYING OUT THE INVENTION

To eliminate the ambiguity of the substance, the present inventors used highly purified murine IFN. For this purpose, large scale production by genetic engineering technique followed by purification of the product is one of good ways. Details of this method have been reported (Murine IFNα: Shaw, G.D. et al., Nucleic Acids Research, 11, 556-573, 1982; Murine IFNβ: Higashi, Y. et al., J. Biol. Chem., 258, 9522, 1983, Senda, T. et al., Proc. Japan Acad., 66, Ser.B,77, 1990; Murine IFNγ: Gray, P.W. and Goeddel, D.V., Proc. Natl. Acad. Sci., 80, 5842, 1983). Needless to say, naturally occurring murine IFN may be used without any problem if it is sufficiently purified. In the examples of the present invention, as the murine IFNα, a commercially available product (Lot. B13010, 10⁶ units/ml/vial, Calbiochem-Novabiochem International, San Diego, CA) was used. As the murine IFNγ, a standard product Lot 254F3, 2.06 x 10⁶ units/ml, provided by Genentech, San Francisco, CA was used. As the murine IFNβ, a product Lot M-0034, 10⁷ units/ml/vial, prepared by Toray Industries, Inc. was used. The process for producing the murine IFNβ by genetic engineering technique as well as the method for purifying the obtained product have been reported in detail (Tanaka, T. et al., J. Interferon Res., 6, 429, 1986, Matsuda, S. et al., J. Interferon Res., 6, 519, 1986). Alternatively, since highly purified murine IFNs are also commercially available from other manufacturers (e.g., Hycult biotechnology, Paesel GmbH, Cosmo Bio and Genzyme), these may also be employed.

To eliminate the ambiguity in biological evaluations, the relative activities to the cells in the bone formation system and the bone resorption system from the same species, especially from the same strain, were quantitatively dealt with, thereby overcoming the drawback in the prior art. That is, even if experimental animals of the same species are used, the response to various hormones largely differ from strain to strain, so that it is necessary to examine the relative effectiveness on the bone resorption system-bone formation system using the same strain. For example, it is known that the rates of decrease in bone volume by ovariectomy in mice which are the model of type I osteoporosis are clearly different depending on the strain (T. Hosaka, ANITEX, Vol. 5, 243, 1992). Thus, the present inventors strictly and quantitatively compared the actions on the osteoblasts responsible for the bone formation system and on the osteoclasts responsible for the bone resorption system using highly purified murine IFN, the osteoblasts and the osteoclasts being originated from the same murine strain.

As a result, IFNβ very strongly inhibits formation of osteoclasts. On the other hand, IFNβ does not substantially inhibit any of the proliferation, differentiation and mineralization of osteoblasts, and IFNβ even promotes the mineralization depending on the conditions. The present inventors revealed these facts for the first time, thereby completing the present invention. The present inventors confirmed the strong inhibition of formation of osteoclasts by IFNγ. However, in addition, the present inventors revealed for the first time that IFNγ strongly inhibits mineralization in the bone formation system in the simple *in vitro* system including osteoblasts alone. This result well explains the above-mentioned result of osteoid formation in mineralization disorder *in vitro* by IFNγ (Mann, G.N. et al., Endocrinology, 135, 1077, 1994). Mineralization is an indispensable process to the completion of bone tissues which are hard tissues. Thus, the present inventors proved that the property of IFNβ is much more preferred to that of IFNγ for the improvement of bone volume, thereby completing the present invention.

Needless to say, the present inventors know that the strong inhibition by IFNβ against the formation of osteoclasts can be effectively utilized for prevention and treatment of bone disorders related to cancer, such as bone metastasis of, for example, mammary carcinoma, lung cancer, prostate cancer, thyroid gland carcinoma, renal cancer, colon cancer, cancer of digestive tract, cancer of esophagus and the like (Stoll, B.A., Bone Metastasis: Monitoring and Treatment, Raven Press, N.Y., 1983). This is because that the formation and development of bone metastatic foci of tumors are intimately related to increase and activation of osteoclasts (Galasco, C.B.S., Skeletal Metastases, Butterworths, pp.22-51, 1986). Therefore, needless to say, IFNβ may be effectively used for therapy of hypercalcemia (a mode of tumor-related bone disorders) accompanied by tumors. The fact that IFN strongly inhibits formation of osteoclasts *in vitro* and, on the other hand, it does not hinder the proliferation, differentiation and mineralization of the cells of the bone formation system or even promotes the mineralization depending on the conditions, and the fact that IFN inhibits *in vivo* the bone resorption and increase the bone volume which is once decreased in osteoporosis model animals clearly show that the relative evaluations in both the bone resorption system and the bone formation system are indispensable. No matter how strong the inhibition by a substance against the bone resorption system may be, this alone does not promise that the substance increases the bone volume. This is apparent from the experiments using IFNγ described here and from the reported results (Mann, G.N. et al., Endocrinology, 135, 1077, 1994). That is, even if the bone resorption-inhibition action by IFN is discovered, unless the action on the bone formation system is not clarified, the invention directed to the use of IFN as a drug for preventing or treating bone disorders is incomplete.

The ambiguity was first overcome by the *in vitro* test in the present invention, and the invention was completed by the proof in the *in vivo* test. Further, IFNβ puts the body forward to relative bone formation, and this action is more prominently exhibited in the body in which the bone volume is abnormal (e.g., ovariectomized mice which are post menopausal osteoporosis models employed in the examples of the present invention) than in the body having the normal bone volume. That is, as described in the examples of the present invention, administration of IFNβ at a dose of 10³ units/head/time to mice which became osteoporosis by ovariectomy exhibited prevention effect against further loss in the bone volume. On the other hand, although the action (bone resorption-inhibition action) inhibiting excretion of urinary deoxypyridinoline (hereinafter referred to as "D-Pyr" for short) is exhibited also in the sham operation groups, this did not immediately bring about sharp increase in the bone volume of the mice. This may be because of the homeostasis which prevents abnormal increase in the bone volume, thereby adjusting the bone volume not to reach the state such as marble bone disease. In fact, the dry bone weight was about the same as that of the control group to which physiological saline was administered. Thus, it was proved that the effect by IFNβ to increase the bone volume is more effectively exhibited in the individuals having abnormally low bone volume (due to disorders or disturbed homeostasis) than in the individuals having the normal bone volume.

These facts clearly indicate that IFNβ is very effective for prevention and therapy of bone disorders which show abnormal bone volume in the whole or a part of the bone tissues, such as metabolic bone disorders, hormonal bone disorders, osteoporosis, bone fracture, alveolar bone disorders. In view of the effect discovered by the present invention, it is apparent that IFN can be used for bone disorders of animals other than human. However, in view of the species specificity of IFN, it is preferred to use the IFN originated from the same species as the animal to be treated. In recent years, the environments for raising companion animals such as dogs and cats have been very much improved. That is, highly advanced medical technologies are applied to the animals and feeds containing well-balanced nutrients are prevailing. As a result, the ages of the companion animals are now rapidly getting higher and higher. The various bone disorders including those in the dental field of the aged animals are surprisingly similar to those of human. Thus, the IFNs from various animals may be used for prevention and treatment of metabolic bone disorders, hormonal bone disorders, osteoporosis, bone fracture, alveolar bone disorders of the corresponding animals.

The IFN used in the present invention may be IFNβ, or consensus type or hybrid type containing the amino acid sequence of IFNβ, and may be one naturally occurring, produced by genetic engineering or by chemical synthesis.

For the production of naturally occurring IFNβ, fibroblasts as well as established cell lines thereof are preferably employed. In cases of producing IFN by genetic engineering technique, as the host cells, mammalian cells such as CHO (Chinese hamster ovary) cells and mouse C127 cells; cells of insects such as silkworm and Barathra; cells of microorganisms such as *E. coli, B. subtilis* and yeasts may be employed. Further, mouse, rat, hamster, rabbit, goat, ovine, swine, bovine and the like may be employed.

The thus prepared IFNβ may be purified by applying the culture supernatant of the cells, insect extract, bacterium extract or body extract to various chromatography. Any chromatography may be employed as long as it has an affinity to IFNβ. For example, columns containing silica or calcium phosphate as an adsorbent; columns having heparin, dye or a hydrophobe as a ligand; metal chelating columns; ion-exchange columns; and gel permeation columns may be employed.

The IFN obtained according to the present invention may be used as a drug as it is or the IFN may be admixed with one or more pharmaceutically acceptable carrier or additive. Examples of these include almost all of the carriers suited for parenteral administration, such as water, physiological saline, Ringer's solution, Hanks' solution and glucose solution, as well as solutions of lactose, dextrose, ethanol, glycerol, albumin and the like. These compositions may contain other additives such as stabilizers, antioxidants, antibacterial agents, antiseptics, buffer agents, surfactants and the like as required. Further, to prevent or relief of flu like symptom, an anti-inflammatory drug may be incorporated in the IFN formulation. The additive or combination of additives may be appropriately selected from those described above depending on the particular formulation of the drug, although, needless to say, the additives are not restricted to those described above.

The administration route is not restricted, and appropriately selected depending on the disorder to be treated. The drug may be administered orally; parenterally or systemically, that is, subcutaneously, intramuscularly, intraperitoneally, intravaginally, intrarectally, or by intravenous injection; and/or intranosally and/or intrapulmonally. Further, a sustained release system of indwelling type such as an osmotic pump (e.g., alzet osmotic pump, Alzet Corporation, Palo Alto, CA) may be selected depending on the disorder to be treated.

When the drug is administered intranosally or intrapulmonally, the drug may be in the form of solution or powder formulated by a medically acceptable device which produces fine solution drops or fine powder. Further, the IFN according to the present invention may be formulated as one for topical application. For example, the drug may be directly infused to the site to be treated, or a carrier of the drug which allows for sustained release of the drug may be directly embedded in the site to be treated. Examples of such a carrier include hydrogels, polylactic acid and collagen matrices. In cases where a collagen matrix originated from an animal other than human is employed, it is preferred to use atelocollagen (such as Zyderm, Collagen Corp., Palo Alto CA) in which the antigenecity of collagen is deleted. Further, hydroxyapatite calcium phosphate (e.g., HA-TCP, Zimmer Inc. Warsaw, IN) which is used for topical reconstruction of bones in the field of orthopedic surgery and dental field, may also be used as an appropriate carrier. Still further, in cases where flu like symptom such as fever is expected or actually occurs, to prevent or to relieve the symptom, an anti-inflammatory drug may also be used in combination with IFN without problem.

The accurate dose of the IFN according to the present invention varies depending on the age, body weight and sex of the patient; as well as on the type, characters and stage of the disorder. Therefore, the accurate dose is not determined beforehand and determined by the physician. However, usually, appropriate dose for systemic administration per day per man is about 10,000 units to about 10,000,000 units, and appropriate dose for topical administration is usually about 10,000 units to about 3,000,000 units.

### Examples

The present invention will now be described by way of examples thereof. It should be noted, however, the examples are presented for the illustration purpose only and the present invention is not restricted to the examples.

### Example 1

### Action on Formation of Osteoclast Like Cells of Mice of ddy Strain

### 1) Method for Preparing and Culturing Bone Marrow Cells

Bone marrow cells were prepared in accordance with the method by Takahashi et al (Takahashi, N. et al., Endocrinology, 122, 1373, 1988). Male mice of ddy strain of 7 weeks old were exanguinated to death under ether anesthesia and the femur and the tibia of hind legs were collected from each mouse. The connective tissues on the surfaces of the bones were carefully removed, and both ends of each bone were cut off with scissors. The bone marrow cells were suspended in α-Minimum Essential Medium (α-MEM, Nissui Pharmaceuticals Co.) containing 10% pre-colostrum new born calf serum (Mitsubishi Chemical, hereinafter referred to as "JCS" for short) and the suspension was flushed out through a 25G needle attached to a syringe to collect the suspension in a centrifugal tube. The collected suspension was washed by centrifugation in the same medium at 2000 rpm at 4°C for 5 minutes. The precipitated cells were dispersed in the above-mentioned medium and the washing by centrifugation was repeated twice, thereby preparing bone marrow cells. Murine IFN-α (hereinafter referred to as "MuIFNα" for short), Lot. B13010, 10⁶ units/ml/vial (Calbiochem-Novabiochem International, San Diego, CA), murine IFN-β (hereinafter referred to as "MuIFNβ" for short), Lot M-0034, 10⁷ units/ml/vial (Toray Industries, Inc.) and murine IFNγ (hereinafter referred to as "MuIFNγ" for short), Lot 254F3, 2.06 x 10⁶ units/ml (Genentech, CA) were respectively dissolved in 10% JCS-containing α-MEM (Nissui Pharmaceuticals Co.) to the desired concentrations. Active vitamin D₃ (1α,25-dihydroxyvitamin D₃, Wako Pure Chemical Industries, Ltd., hereinafter referred to as "Vit D₃" for short) was dissolved in argon-saturated ethanol, and the obtained solution was diluted so as to attain the final ethanol concentration of 0.1% in all of the culture systems.

### 2) Evaluation of Ability to Form Osteoclasts

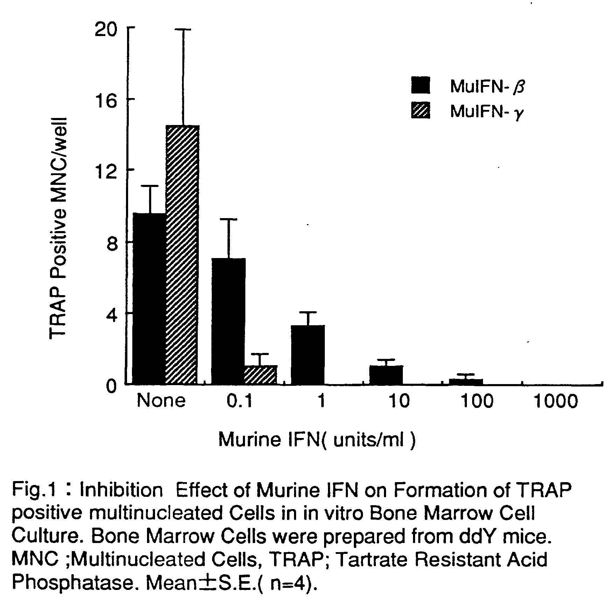

The number of viable bone marrow cells was counted using tripam blue dye exclusion test and the bone marrow cells were diluted with α-MEM containing 10% JCS, 10⁻⁸M Vit D₃, 10⁻⁷ M dexamethasone to a population density of 1.5 x 10⁶ cells/ml, followed by placing the resulting suspension in each well in 24-well plates in an amount of 0.45 ml/well. To each well, 50 µl of the medium containing varying concentration of IFN was added to make the volume to 0.5 ml/well and culturing was begun. At every three days, 0.4 ml of the culture medium was removed from each well and 0.4 ml of fresh medium (the above-mentioned medium containing the respective concentration of IFN) was added. On Day 8 from the beginning of the culturing, the medium was removed and the cells were fixed with mixed solution of ethyl alcohol and diethyl ether (1:1) and the cells were stained with tartrate resistant acid phosphatase (TRAP), followed by counting TRAP positive multinuclear cells with a phase contrast microscope. Fig. 1 shows the results. Both the MuIFNβ and MuIFNγ added to each of the above-mentioned system strongly inhibited formation of osteoclast like cells. Each column in the figure shows the average and standard error of 4 cases. Clear dose dependence was observed in both IFNs. The inhibition effect of IFNγ was stronger than that of IFNβ. That is, the inhibition concentration IC₅₀ of IFNγ was not more than 0.1 units/ml, and that of IFNβ was 0.2 units/ml.

### Example 2

### Action on Formation of Osteoclast Like Cells of Mice of C 57 BL/6 Strain

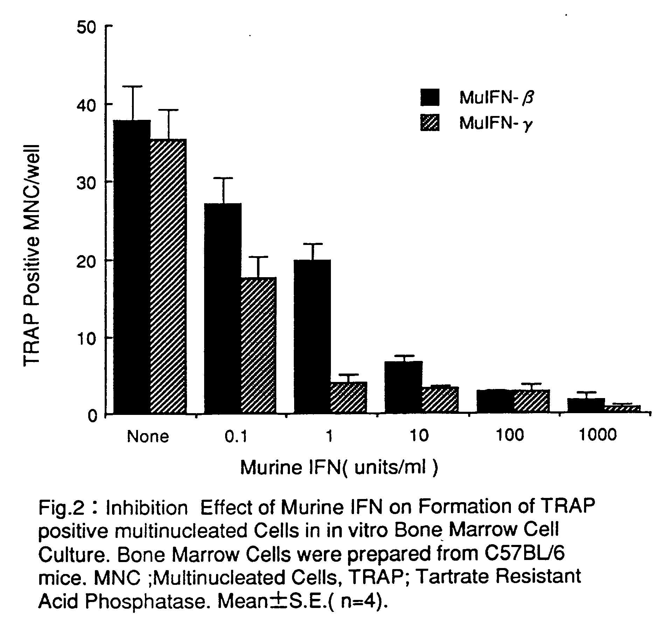

In place of mice of ddy strain, male mice of C 57 BL/6 of 7 weeks old were used. The mice were exsanguinated to death under ether anesthesia and the femur and the tibia of hind legs were collected from each mouse. The connective tissues on the surfaces of the bones were carefully removed, and both ends of each bone were cut off with scissors. The bone marrow cells were suspended in α-MEM containing 10% JCS and the suspension was flushed out through a 25G needle attached to a syringe to collect the suspension in a centrifugal tube. The collected suspension was washed by centrifugation in the same medium at 2000 rpm at 4°C for 5 minutes. The precipitated cells were dispersed in the above-mentioned medium and the washing by centrifugation was repeated twice. Formation of osteoclast like cells was measured by the same method as in Example 1. As a result, as in the case of mice of ddy strain, both MuIFNβ and MuIFNγ strongly inhibited formation of osteoclast like cells from bone marrow cells of mice of C 57 BL/6 strain. Fig. 2 shows the results. The IC₅₀ values of formation inhibition of osteoclast like cells of MuIFNβ and MuIFNγ were 0.1 units/ml and 1.5 units/ml, respectively. Each column in the figure shows the average and standard error of 4 cases.

### Example 3

### Action on Proliferation of Murine Osteoblasts (in vitro single administration)

Murine osteoblast like cells MC3T3-E1 originated from C 57 BL/6 mouse (Kodama et al., Jap. J. Oral Biol., 23, 899, 1982) were washed with PBS(-) and the cells were dissociated by incubation in PBS(-) containing 0.1% pronase (Actinase E, Kaken Pharmaceuticals Co.), followed by dispersing the cells in α-MEM containing 10% JCS and 50 µg/ml of L-ascorbic acid (ASA). The ability to promote proliferation of cells was evaluated as follows. That is, MC3T3-E1 cells were placed in each well in 24-well plates (Corning) in an amount of 5000 cells/well. Then each IFN dilution was added to each well in an amount of 0.1 ml/well, and the culturing was begun. On Day 2, the medium was removed and the each IFN dilution was added in an amount of 0.5 ml/well (total amount of liquid: 0.5 ml/well). On Day 6, the medium was removed and the cells were washed with PBS(-) and a mixed enzyme solution of 0.1% pronase/0.1% collagenase was added to each well in an amount of 200 µl/well, followed by allowing to react at 37°C for 15 minutes. Then α-MEM containing 10% fetal calf serum (Gibco, hereinafter referred to as "FCS" for short) was added in an amount of 300 µl/well to disperse the cells therein and the number of the cells was counted with a Coulter counter.

The actions on cell proliferation are shown in Fig.' 3. Each column in the figure shows the average and standard error of 4 cases. Both murine IFNs showed very weak or substantially no inhibition actions on the proliferation of MC3T3-E1 cells. Although slight inhibition of proliferation was observed with MuIFNγ from the dose of 1 unit/ml, the dose dependence is small, and the inhibition was only about 30% even at a concentration as high as 10,000 units/ml. On the other hand, MuIFNβ did not inhibit the proliferation at all even at 10,000 units/ml.

### Example 4

### Actions on Differentiation Ability and Mineralization of Murine Osteoblasts (in vitro single administration)

As the parameters for evaluation of differentiation ability, alkaline phosphatase (ALPase) and mineralization were employed. Detection of alkaline phosphatase which is a marker of differentiation of osteoblasts was carried out on Day 8 from the beginning of the culturing by staining method. Evaluation of mineralization was carried out as follows. That is, MC3T3-E1 cells were placed in each well of 24-well plates in an amount of 50,000 cells/ml (10 times in the case of anti-cell test in Example 3). On Day 4, after confirming that the cells sufficiently reached confluency, the medium was discarded and fresh α-MEM containing 10% JCS, 50 µg/ml of L-ascorbic acid and 10 mM β-glycerophosphate was added in an amount of 0.9 ml/well, followed by adding medium containing varying concentration of IFN. Four days later, the medium was replaced with IFN-free medium, and the medium was replaced with IFN-free medium every 4 days. On Day 14, Von Kossa stain was performed to visualize the calcified foci. Expression of alkaline phosphatase activity which is a differentiation marker is an indispensable step to the subsequent mineralization. Both murine IFNs did not inhibit expression of the enzyme activity which is a differentiation marker.

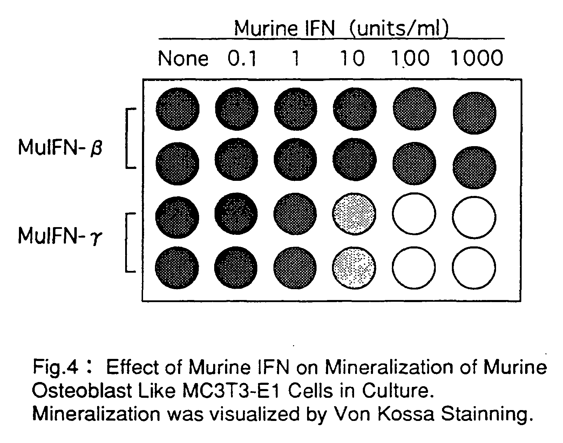

On the other hand, a large difference was observed between MuIFNβ and MuIFNγ in the mineralization process as shown in Fig. 4. That is, while mineralization proceeded without substantial influence even in the presence of IFNβ, IFNγ showed strong and dose dependent inhibition action on mineralization. The inhibition effect was expressed at a concentration as low as 10 units/ml, and IC₅₀ value of inhibition of mineralization was about 3-4 units/ml. Needless to say, for complete repair of the bone tissues as hard tissues, the inhibition of mineralization by MuIFNγ is not preferred, so that the superiority of IFNβ to IFNγ is apparent.

### Example 5

### Quantification of Calcium and Inorganic Phosphorus in Calcified Foci of Murine Osteoblasts MC3T3-E1

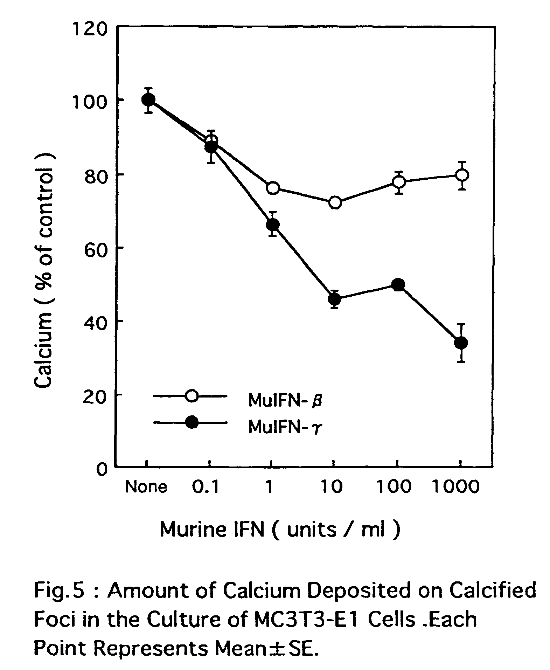

MC3T3-E1 cells were placed in each well in 24-well plates in an amount of 50,000 cells/well and cultured to confluency. The medium was removed and fresh α-MEM containing 10% JCS, 50 µg/ml of L-ascorbic acid and 10 mM β-glycerophosphate was added in an amount of 0.9 ml/well, followed by adding 0.1 ml/well of medium containing varying concentration of IFN. The medium was replaced with IFN-free medium every 4 days. On Day 14, the medium was removed and the cells were lightly washed with Hank's solution. Anhydrous ethanol was added in an amount of 1 ml/well and fixation was carried out at room temperature for 15 minuets. Then the anhydrous ethanol was replaced with fresh anhydrous ethanol in an amount of 1 ml/well and 5 minutes later, each well was dried in the air. Then IN hydrochloric acid was added in an amount of 1 ml/well and extraction was carried out at room temperature for 30 minutes. The total contents of each well were transferred to an Eppendorf tube and centrifuged at 1800 x g at 4°C for 10 minutes. The amount of calcium in the supernatant was measured by Calcium C Test Wako^{®} and the amount of inorganic phosphorus in the supernatant was measured by Phospha B Test Wako^{®}. Although slight inhibition of calcium deposition was observed with the addition of IFNβ, the degree was small and the calcium deposition was only 20% smaller than the control even at a concentration of 1000 units/ml. On the other hand, IFNγ exhibited dose dependent and strong inhibition of mineralization and the calcium deposition was as much as 70% smaller than the control at a concentration of 1000 units/ml. The IC₅₀ ' value was as low as about 10 units/ml (Fig. 5). Each point in the figure shows the average and standard error of 4 cases.

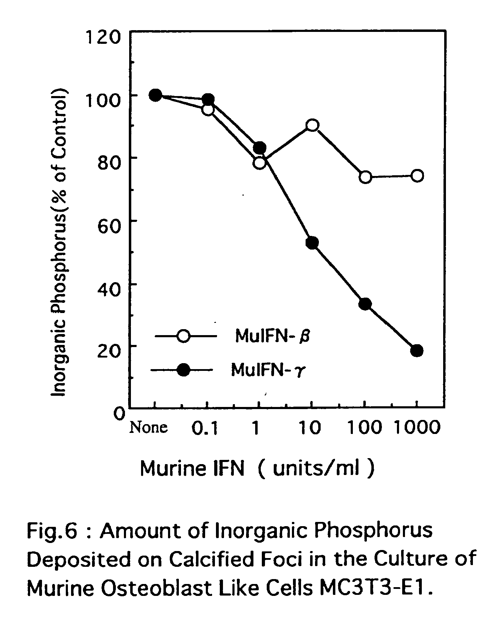

The amounts of inorganic phosphorus extracted from calcified foci are shown in Fig. 6. Each point in the figure shows the average of 4 cases. The amounts of the extracted inorganic phosphorus showed good correlation with the amounts of calcium. The amount of the extracted inorganic phosphorus for 1000 units/ml of IFNβ was only about 20% smaller than the control and IC₅₀ value could not be obtained. On the other hand, the amount of the extracted inorganic phosphorus for 1000 units/ml of IFNβ was about 80% smaller than the control and the IC₅₀ value was about 10 units/ml, which is extremely small (Fig. 6).

### Example 6

### Actions on Differentiation Ability and Mineralization of Murine Osteoblasts (in vitro multiple administration)

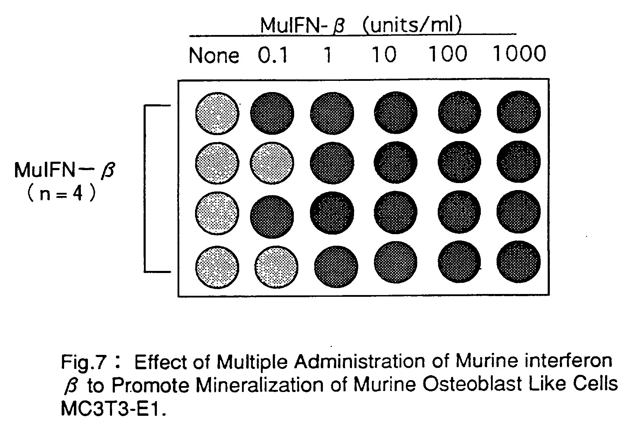

MC3T3-E1 cells were placed in each well in 24-well plates in an amount of 30,000 cells/well and cultured to confluency. The medium was removed and fresh α-MEM containing 10% JCS, 50 µg/ml of L-ascorbic acid and 10 mM β-glycerophosphate was added in an amount of 0.9 ml/well, followed by adding 0.1 ml/well of medium containing varying concentration of IFN. The medium was replaced with the medium containing the respective concentration of IFN every 4 days. On Day 9 from the first treatment with IFN, Von Kossa stain was performed to visualize the calcified foci. As a result, unlike the case of single administration, by the twice administration of IFN, the number of calcified foci and the intensity of staining were dose dependently increased (Fig. 7).

### Example 7

### Therapeutic Effect of Murine IFNβ Against Osteoporosis in Model Mice

Female mice of ddy strain of 8 weeks old were divided into 5 groups and experiment was carried out. Bilateral ovariectomy was performed on the mice in 3 groups and sham operation alone was performed on the mice in 2 groups. Thereafter, the mice were raised with normal diet for 1 month, thereby making the state of osteoporosis in the ovariectomized groups. In this model, it is known that the mice become the state of osteoporosis 2 to 3 weeks after the ovariectomy (T. Hosaka et al., ANITEX, Vol. 5, 243, 1992; Miyaura, C. et al., J. Bone Mineral Res., 10, 1365, 1995). From the third weeks from the ovariectomy, murine IFN was subcutaneously administered (100 µl/head/time) once per day, totally 25 times. On Day 30 from the beginning of administration, under ether anesthesia, body weight was measured, blood was collected, and femur was collected and dry bone weight thereof was measured for each mouse.

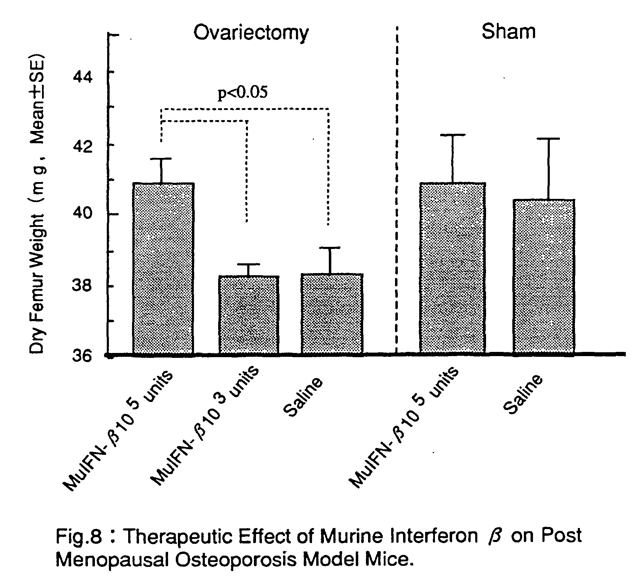

Drying of femur was performed as follows. That is, femur was fixed with 2 ml of 70% ethanol per one taken out femur at room temperature for 24 hours and then the attached muscles and connective tissues were carefully removed with a forceps and surgical knife with replaceable blade. After immersing the femur in fresh 70% ethanol for 72 hours at room temperature, the femur was dried in a hot wind-generating incubator (Koukensha Engineering Co., Tokyo) at 55°C for 24 hours. The weight of the dried bone was measured with an electronic force balance (Mettler AE50, Metteler Instrumente AG, Switzerland). Murine IFN to be administered was diluted to the desired concentration with physiological saline. During the test, urine of mice was collected with metabolic cage (KN-645, Natsumeseisakusho, Tokyo) and frozen to store. To one group subjected to the sham operation, physiological saline (Otsuka Pharmaceutical Co.) was administered, and to another group subjected to the sham operation, murine IFNβ was administered at a dose of 10⁵ units/head/time (diluted with physiological saline when use). To one group subjected to ovariectomy, physiological saline was administered and to the other two groups subjected to ovariectomy, MuIFNβ was administered at doses of 10³ units/head/time and 10⁵ units/head/time, respectively. Fig. 8 shows the dry bone weights. Ovariectomy which is a model of post menopausal osteoporosis caused clear decrease in bone volume in mice. However, administration of MuIFNβ at a dose of 10⁵ units/head/time recovered the normal level of bone volume, so that MuIFNβ exhibited clear therapeutic effect. On ' the other hand, in the groups subjected to the sham operation, significant increase in the bone volume was not observed even at a dose of 10⁵ units.

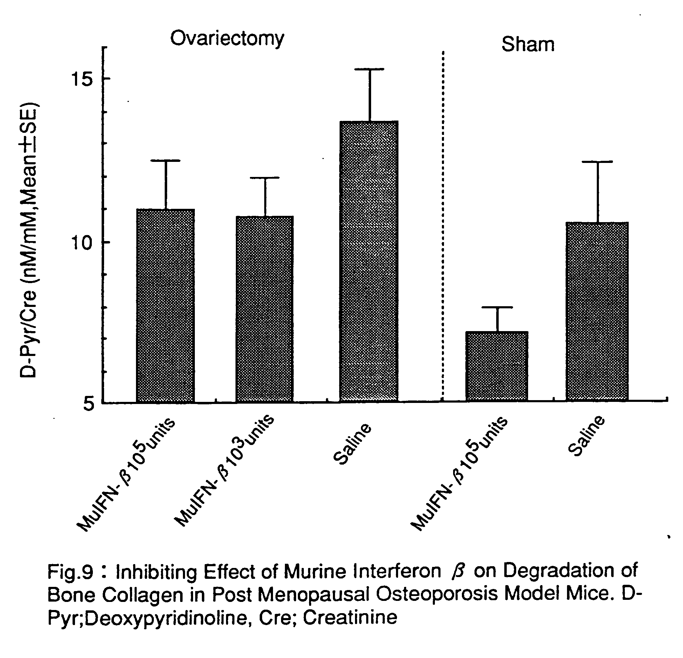

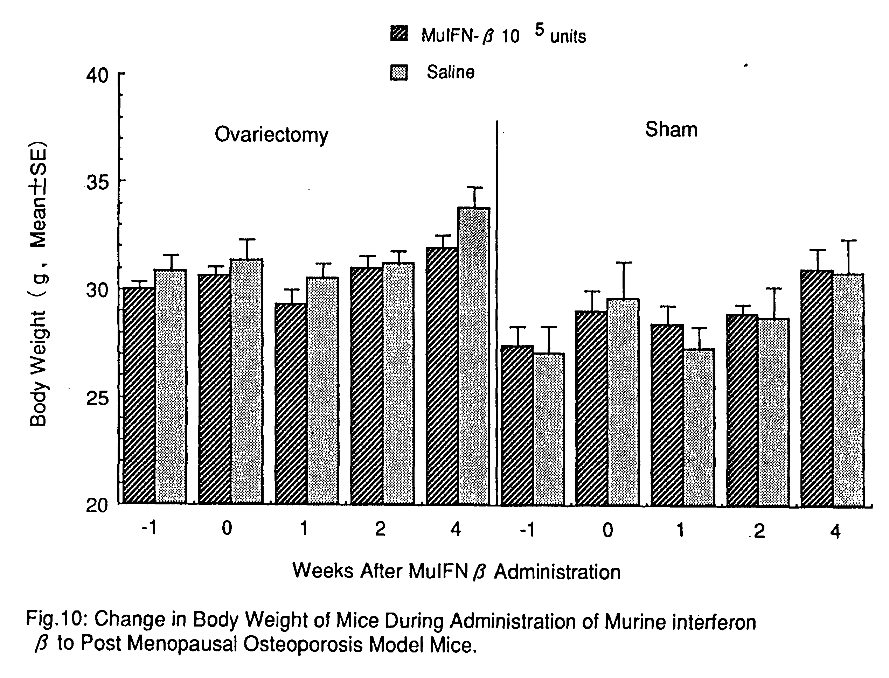

It is well-known that the increase and decrease in the amount of urinary D-Pyr excreted in urine, which is a crosslinked substance of bone collagen, is an excellent marker reflecting the degree of degradation of bone collagen, that is, the degree of bone resorption (C.P. Jerome et al., Bone and Mineral, 19, 117-125, 1992). For measurement of urinary D-Pyr, a commercially available kit (PYRILINKS - D Assay, METRA BIOSYSTEMS) was used. The amount of D-Pyr of mice in each group compensated with the amount of the urinary creatinine in Week 4 from administration of MuIFNβ is shown in Fig. 9. In the group to which physiological saline was administered, the amount of D-Pyr which is a bone resorption marker clearly increased, while in the groups to which MuIFNβ was administered at doses of 10³ units/head/time and 10⁵ units/head/time, respectively, the amount of excreted D-Pyr was smaller, that is, inhibition of bone resorption was observed. The degree of inhibition was about the same level as in the group subjected to sham operation and administered with physiological saline, that is, about the same level as the normal value in urine. Fig. 10 shows the change in body weight before and after beginning of administration of MuIFNβ. Although not shown in the figure, the body weight of the mice immediately before the ovariectomy (3 weeks before the commencement of administration of MuIFNβ) was 25.6±0.18 g (n=33) and that of the group to be subjected to sham operation was 25.7±0.36 g (n=11), so that the body weights of the groups were about the same. Although at 2 weeks after the surgery, that is, at 1 week before commencement of administration of IFN, the body weights of the groups were different, this reflects the increase in body weight due to ovariectomy (Miyaura, C. et al., J. Bone Mineral Res., 10, 1365, 1995). By autopsy after the test, large decrease in uterine weight in the ovariectomized group was confirmed. This is also a typical response due to ovariectomy (Miyaura, C. et al., J. Bone Mineral Res., 10, 1365, 1995). Administration of IFNβ continuously for 4 weeks at a dose exhibiting the therapeutic effect, that is, at a dose at which the bone volume is increased, no problematic change in body weight was observed in any group.

### Example 8

### Comparison of Effects of Various Murine IFNs on Inhibition of Formation of Murine Osteoclasts

The effects of the three murine IFNs, that is, MuIFNβ, γ and α in terms of abilities to inhibit formation of osteoclast like cells from bone marrow cells in mice of ddy strain were compared. The test was carried out as in Example 1. All of the murine IFNs inhibited the formation of osteoclast like cells, and the effect was strong in the order of γ, β and α. The IC₅₀ values determined from the dose dependence curves thereof were not more than 0.1 unit/ml for IFNγ and about 0.9 units/ml for IFNβ while that for IFNα was 22.4 units/ml. Thus, the activity of IFNα to inhibit formation of osteoclasts was about 1/20 of that of IFNβ and about not more than 1/100 of that of IFNγ.

### INDUSTRIAL AVAILABILITY

As described above, by the present invention, the relative action to promote bone formation by IFNβ was proved in *in vitro* tests and in animal tests, so that the usefulness of IFNβ as a drug for treating and preventing bone disorders was shown. Especially, the discovery that the recovery of the bone volume by IFNβ *in vivo* is more prominent in the individuals having abnormal bone volumes than in the individuals having normal bone volumes provides a high advantage to the industrial use of IFNβ as a drug for treating bone disorders.

Further, the possibility to use interferon inducers as drugs for treating bone disorders was discovered, which provides a high advantage to the industrial use of IFNβ as a drug for treating bone disorders.

## Claims

1. Use of interferon β for the manufacture of a drug for treatment of bone disorder disturbed relative balance between bone resorption and hone formation, wherein the bone disorder is osteoporosis, metabolic bone disorder, hormonal bone disorder, periodontal disease-related bone disorder, bone fracture, or hypercalcemia.

2. The use of claim 1, wherein said interferon β is a naturally occurring, consensus type, hybrid type containing the amino acid sequence of interferon β, or a recombinant type.

3. The use of claim 1 wherein said osteoporosis is postmenopausal osteoporosis.

4. The use according to claim 1, wherein said metabolic bone disorder is selected from the group consisting of Paget's disease and osteoarthritis.

5. The use according to claim 1, wherein said hormonal bone disorder is Cushing's syndrome or hyperparathyroidism.

6. The use according to claim 1, wherein said periodontal disease-related bone disorder is alveolar bone disorder.

## Patentansprüche

1. Verwendung von Interferon β für die Herstellung eines Medikaments zur Behandlung einer Knochenfunktionsstörung, wobei die das relative Gleichgewicht zwischen Knochenresorption und Knochenbildung gestört ist, worin die Knochenfunktionsstörung eine Osteoporose, eine metabolische Knochenfunktionsstörung, hormonelle Knochenfunktionsstörung, Parodontalerkrankungs-verwandte Knochenfunktionsstörung, eine Knochenfraktur oder Hyperkalzämie ist.

2. Verwendung nach Anspruch 1, worin das besagte Interferon β ein natürlich vorkommendes, ein Konsensustyp, ein Hybridtyp, der die Aminosäuresequenz von Interferon β enthält, oder ein rekombinanter Typ ist.

3. Verwendung nach Anspruch 1, worin die besagte Osteoporose eine postmenopausale Osteoporose ist.

4. Verwendung nach Anspruch 1, worin die besagte metabolische Knochenfunktionsstörung ausgewählt ist aus der Gruppe, bestehend aus der Paget'schen Krankheit und Osteoarthritis.

5. Verwendung nach Anspruch 1, worin die besagte hormonelle Knochenfunktionsstörung ein Cushing-Syndrom oder Hyperparathyroidismus ist.

6. Verwendung nach Anspruch 1, worin die besagte Parodontalerkrankungs-verwandte Knochenfunktionsstörung eine alveolare Knochenfunktionsstörung ist.

## Revendications

1. Utilisation d'interféron β pour la fabrication d'un médicament destiné au traitement d'une maladie osseuse due à une perturbation de l'équilibre relatif entre résorption osseuse et formation osseuse, la maladie osseuse étant l'ostéoporose, une maladie osseuse d'origine métabolique, une maladie osseuse d'origine hormonale, une maladie osseuse associée à une parodontopathie, une fracture osseuse ou une hypercalcémie.

2. Utilisation selon la revendication 1, où ledit interféron β est un interféron β de type courant, naturel, de type hybride contenant la séquence d'acides aminés de l'interféron β ou de type recombinant.

3. Utilisation selon la revendication 1, où ladite ostéoporose est une ostéoporose post-ménopausique.

4. Utilisation selon la revendication 1, où ladite maladie osseuse d'origine métabolique est sélectionnée parmi le groupe constitué par la maladie de Paget et une ostéoarthrite.

5. Utilisation selon la revendication 1, où ladite maladie osseuse d'origine hormonale est le syndrome de Cushing ou l'hyperparathyroïdie.

6. Utilisation selon la revendication 1, où ladite maladie osseuse associée à une parodontopathie est une maladie osseuse alvéolaire.
